# EUROPEAN PATENT APPLICATION

(11) **EP 4 108 233 A1**
(43) Date of publication of application: **28.12.2022**
(21) Application number: 21386036.4
(22) Date of filing: 24.06.2021
(51) Int. Cl.: A61K 9/08, A61K 31/137, A61K 47/10

(54) **ORAL SOLUTION COMPRISING A CINACALCET SALT**

(71) Applicant: Faran S.A., 14564 Nea Kifissia (GR)
(72) Inventor: Koukoulommatis, Panagiotis, 15238 Patima II Chalandriou (GR)
(74) Representative: Roukounas, Dimitrios

(57) **Abstract**

Oral pharmaceutical solution comprising a pharmaceutically acceptable cinacalcet salt and a pharmaceutically acceptable carrier comprising a mixture of propylene glycol and a polyethylene glycol, or a mixture of two polyethylene glycols, a polyol, a polymer selected from polyvinylpyrrolidone and/or copovidone, water, and a buffer, wherein the pH of the solution is from 4.0 to 7.5.

## Description

### TECHNICAL FIELD

The present invention relates to oral pharmaceutical solutions comprising a pharmaceutically acceptable cinacalcet salt.

### BACKGROUND OF THE INVENTION

Cinacalcet is a calcimimetic agent (i.e., it mimics the action of calcium on tissues) that increases the sensitivity of the calcium-sensing receptor to activation by extracellular calcium, that was first disclosed in the patent application number EP0724561 by NPS Pharmaceuticals. The patent application also discloses the usage of cinacalcet and its pharmaceutically acceptable salts, for the treatment of primary or secondary hyperparathyroidism.

Cinacalcet has the chemical name N-[(1R)-1-naphthalen-1-ylethyl]-3-[3-(trifluoromethyl)phenyl]propan-1-amine and the following structural formula (Formula I):

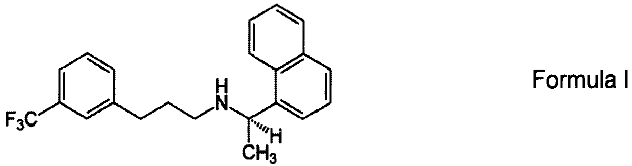

Cinacalcet is commercially available in particular in hydrochloride salt form. Cinacalcet hydrochloride is a white to off-white, crystalline solid that is soluble in methanol or 95 percent ethanol and slightly soluble in water.

The commercial cinacalcet hydrochloride tablet formulations are marketed under the trade name Sensipar^{®} in the United States and Australia, and as Mimpara^{®} in Europe. These tablets are formulated as film-coated, oval-shaped tablets for oral administration in strengths of 30 mg, 60 mg, and 90 mg of cinacalcet hydrochloride as the free base equivalent (33 mg, 66 mg, and 99 mg as the hydrochloride salt, respectively).

Although oral solid dosage forms such as tablets are very popular mainly due to ease of management, for certain users (e.g. children and the elderly) these forms are not necessarily a convenient option, especially due to difficulty in swallowing these forms. This lack of convenience results in high incidence of non-compliance and ineffective therapy.

Moreover, the Patient Information Leaflet (PIL) of Mimpara^{®} tablets discloses a dosing scheme for children over 3 to less than 18 years of age according to which dosing is adjusted to their body weight, whereas the corresponding adult-dosing and elderly-dosing is subjected to titration dependent on their response. The concept of tailored treatment sets as prerequisite pharmaceutical forms, such as oral solutions, that enable dose fractioning.

Hence, as a means of both ensuring flexibility in dose titration and advanced patient compliance, the development of oral liquid dosage forms of cinacalcet, which can allow optimal dose selection is definitely an existing need.

However, the desire for the development of an oral liquid dosage form that comprises cinacalcet is complicated by the fact that cinacalcet and its pharmaceutically acceptable salts are only slightly soluble in water (e.g. the solubility of cinacalcet hydrochloride in water is estimated to be 0.092 mg/L at 25°C) and the fact that they possess an extremely bitter taste.

According to patent application EP 3241549 cinacalcet is known to possess a bitter taste and it induces numbness of the tongue. As a solution to this problem, taste masked sprinkle compositions of cinacalcet comprising coated cores, to provide enhanced palatability and improved patient compliance, are described therein.

Patent application WO 2019/186516 pertains to ready to use, liquid dosage forms of cinacalcet or pharmaceutical acceptable salts thereof comprising one or more excipients or additives. Although WO 2019/186516 mentions cinacalcet solutions for oral administration in general, it does not disclose any solution compositions. Instead, due to the poor solubility of cinacalcet and its salts, a ready to use suspension dosage form is proposed, as an alternative to the marketed Sensipar^{®} film coated tablets.

However, many drawbacks are known to be related to oral suspensions. For example, when a suspension of an active substance is used there is the problem that the homogeneity of the suspension has to be ensured for a sufficient length of time during removal from the primary packaging (e.g. glass bottle, 100 ml) to ensure accurate dosing. Moreover, the caking caused by contact of the individual particles under the effects of gravity during storage of the suspension is very often difficult to be avoided.

Further to that it has also been found that an unexpected high viscosity/thixotropy, e.g. high viscosity and insufficient flow behavior as well as a brownish discoloration may appear in the cinacalcet hydrochloride suspensions of the prior art, after long-term storage. The insufficient flow behavior becomes apparent in the form of "lumps" which can only be re-dispersed with difficulty by a very vigorous shaking.

The present invention addresses the problems of the prior art knowledge by advantageously providing oral pharmaceutical solutions comprising a pharmaceutically acceptable cinacalcet salt.

### SUMMARY OF THE INVENTION

The present invention is directed to an oral pharmaceutical solution comprising a pharmaceutically acceptable cinacalcet salt in association with a pharmaceutically acceptable aqueous carrier.

The oral pharmaceutical solution according to the invention comprises a pharmaceutically acceptable cinacalcet salt as active ingredient and a pharmaceutically acceptable carrier comprising a mixture of propylene glycol and a polyethylene glycol, or a mixture of two polyethylene glycols, a polyol, a polymer which is polyvinylpyrrolidone and/or copovidone, water, and a buffer, wherein the pH of the solution is from 4.0 to 7.5.

The oral pharmaceutical solution according to the present invention provides the best alternative over conventional capsule, film coated tablet or chewable tablet dosage forms. Apart from achieving better patient compliance, the oral solution of the present invention offers unique advantages such as more reproducible bioavailability and an option of a flexible dosing regimen based on body weight or body surface area.

The present invention provides many benefits in comparison to oral suspension dosage forms. It has the advantage that it provides a physicochemically stable oral pharmaceutical solution of a pharmaceutically acceptable cinacalcet salt, by inhibiting precipitation that typically occurs after extended storage, whereas at the same time it presents excellent organoleptic characteristics.

Further to that, the present invention provides palatable oral pharmaceutical solutions comprising a pharmaceutically acceptable cinacalcet salt that effectively taste mask the bitterness of the active ingredient and at the same time avoids the "cloying" sensation in the mouth, which is a common problem with oral suspensions.

### DETAILED DESCRIPTION OF THE INVENTION

The present invention provides an oral pharmaceutical solution comprising a pharmaceutically acceptable cinacalcet salt as active ingredient.

The inventors' early research confirmed that cinacalcet hydrochloride is an intensely bitter drug by calculating a bitterness value of much more than 50,000, employing an adult sensory panel of 8 persons and following the guidelines described in European Pharmacopoeia (Ph. Eur. 10 - paragraph 2.8.15). According to this method the bitterness value is the reciprocal of the dilution of a compound, a liquid or an extract that still has a bitter taste. It is actually determined by comparison with quinine hydrochloride, the bitterness value of which is set at 200,000.

Numerous combinations of cosolvents and surfactants such as glycerol, propylene glycol, polyethylene glycol 300 or 400, polysorbate 20, 40, 60 or 80, povidone K25, K30, K29/32, 90F, liquid sorbitol, mannitol, sodium lauryl sulfate, copovidone, hydroxypropyl cellulose, hydroxypropyl methylcellulose, poloxamer 20, 40, 60, fructose, sucrose and glucose, were evaluated but did not provide always the desired solubility and/or palatability. It has been found that when surfactants and/or cosolvents were added to cinacalcet hydrochloride solutions, their effect on the solubility and palatability is strongly depended on the combination of the surfactant and the cosolvent and their concentrations.

After intensive testing it has surprisingly been found that only when a pharmaceutically acceptable salt of cinacalcet is combined with certain excipients, it is possible to manufacture an oral solution instead of a suspension of a cinacalcet salt having improved organoleptic properties so that its use by patients, e.g., children or adults having difficulties in swallowing tablets, is highly facilitated.

The oral pharmaceutical solution according to the invention comprises a pharmaceutically acceptable cinacalcet salt as active ingredient and a pharmaceutically acceptable carrier comprising
a) a mixture of propylene glycol and a polyethylene glycol having an average molecular weight from 150 to 800, or a mixture of two polyethylene glycols having an average molecular weight from 150 to 800,
b) a polyol selected from glycerol, sorbitol, mannitol, maltitol, xylitol, erythritol, isomalt, lactitol, polyvinyl alcohol, or a mixture thereof,
c) a polymer which is polyvinylpyrrolidone having an average molecular weight from 20,000 to 1,500,000, and/or copovidone having an average molecular weight from 45,000 to 70,000,
d) water and
e) a buffer,
wherein the pH of the solution is from 4.0 to 7.5.

Preferably, the polyethylene glycol according to the invention is polyethylene glycol 200 (also known as PEG 200), polyethylene glycol 300 (also known as PEG 300), polyethylene glycol 400 (also known as PEG 400), polyethylene glycol 540 (also known as PEG 540-blend) and polyethylene glycol 600 (also known as PEG 600). More preferably, the polyethylene glycol is polyethylene glycol 400.

Preferably, the polyol is selected from glycerol, sorbitol, mannitol, maltitol, xylitol, or amixture thereof. More preferably, the polyol is sorbitol.

Polyvinylpyrrolidone (also known as povidone) is obtained by free-radical polymerization of vinylpyrrolidone in water or 2-propanol and is available in different average molecular weights. The higher the average molecular weight, the greater the viscosity and consequently the adhesive strength. The K-value denotes the intrinsic viscosity of the polymer related to the average molecular weight, and is derived from the relative viscosity of the aqueous solution measured at 25°C. The direct correlation between the average molecular weight and properties enables the appropriate grade to be used in each formulation in the appropriate concentration in order to achieve the optimum effect. Examples of preferred according to the invention commercially available povidone grades include;
Povidone K25 (trade name: Kollidon^{®} 25, manufacturer: BASF, or trade name: Plasdone^{®} K-25, manufacturer: ISP),
Povidone K30 (trade name: Kollidon^{®} 30, manufacturer: BASF, or trade name: Plasdone^{®} K-29/32, manufacturer: ISP),
Povidone K90 (trade name: Kollidon^{®} 90F, manufacturer: BASF, or trade name: Plasdone^{®} K-90, manufacturer: ISP).

The European Pharmacopoeia (Ph.Eur.) and the United States Pharmacopoeia (USP) describe copovidone as a copolymer of 1-ethenylpyrrolidin-2-one and ethenyl acetate in the mass proportion of 3:2, which is readily soluble in water. Examples of preferred according to the invention commercially available copovidone grades include;
Copovidone K25-31 (trade name: Kollidon^{®} VA 64, manufacturer: BASF),
Copovidone K25-31 (trade name: Plasdone^{®} S 630, manufacturer: Ashland).

Preferably, the polymer according to the invention is povidone K25, povidone K90 or copovidone K25-31. More preferably, the polymer is povidone K90.

Any pharmaceutically acceptable system which acts as a buffer in the pH region of the invention can be used in the oral pharmaceutical solution according to the invention. Examples of a buffering agent include but are not limited to ascorbic acid, acetic acid, tartaric acid, citric acid monohydrate, sodium citrate, potassium citrate, acetic acid, sodium acetate, sodium hydrogen phosphate, sodium dihydrogen phosphate, calcium hydrogen phosphate, calcium dihydrogen phosphate, or a mixture thereof.

The oral pharmaceutical solution according to the invention may comprise any pharmaceutically acceptable salt of cinacalcet. Preferably, the pharmaceutically acceptable salt according to the invention is oxalate, or di-p-toluoyl-L-tartrate, or hydrochloride. More preferably, the pharmaceutically acceptable salt according to the invention is hydrochloride.

Preferably, the oral pharmaceutical solution according to the invention comprises from 8 mg/ml to 18 mg/ml of a pharmaceutically acceptable salt of cinacalcet. More preferably, the solution comprises from 10 mg/ml to 16 mg/ml of a pharmaceutically acceptable salt of cinacalcet. Even more preferably, the solution comprises from 12 mg/ml to 14 mg/ml of a pharmaceutically acceptable salt of cinacalcet.

Preferably, the oral pharmaceutical solution according to the invention comprises from 8 mg/ml to 18 mg/ml of cinacalcet hydrochloride. More preferably, the solution comprises from 10 mg/ml to 16 mg/ml of cinacalcet hydrochloride. Even more preferably, the solution comprises from 12 mg/ml to 14 mg/ml of cinacalcet hydrochloride.

Preferably, the concentration of the mixture of propylene glycol and polyethylene glycol having an average molecular weight from 150 to 800, or of the mixture of the two polyethylene glycols having an average molecular weight from 150 to 800 in the oral pharmaceutical solution according to the invention is from 200 to 600 mg/ml. More preferably, the concentration of the mixture of propylene glycol and polyethylene glycol having an average molecular weight from 150 to 800, or of the mixture of the two polyethylene glycols having an average molecular weight from 150 to 800 is from 250 mg/ml to 550 mg/ml. Even more preferably, the concentration of the mixture of propylene glycol and polyethylene glycol having an average molecular weight from 150 to 800, or of the mixture of the two polyethylene glycols having an average molecular weight from 150 to 800 is from 300 mg/ml to 500 mg/ml.

Preferably, the polyol concentration in the oral pharmaceutical solution according to the invention is from 200 to 500 mg/ml. More preferably, the polyol concentration is from 200 mg/ml to 450 mg/ml. Even more preferably, the polyol concentration is from 200 mg/ml to 400 mg/ml.

Preferably, the concentration of the polymer in the oral pharmaceutical solution according to the invention is from 1 to 10 mg/ml. More preferably, the concentration of the polymer is from 2 mg/ml to 9 mg/ml. Even more preferably, the concentration of the polymer is from 3 mg/ml to 8 mg/ml.

The term "mg/ml" used in the present description and claims means mg of the active ingredient or of the excipient(s) per 1 ml of the oral pharmaceutical solution.

Preferably, the pH of the solution is from 5.0 to 6.5.

According to a preferred embodiment, the oral pharmaceutical solution according to the invention comprises from 8 mg/ml to 18 mg/ml of a pharmaceutically acceptable salt of cinacalcet, and a pharmaceutically acceptable carrier comprising
a) a mixture of propylene glycol and polyethylene glycol having an average molecular weight from 150 to 800, or a mixture of two polyethylene glycols having an average molecular weight from 150 to 800, wherein the concentration of the mixture is from 200 mg/ml to 600 mg/ml,
b) a polyol selected from glycerol, sorbitol, mannitol, maltitol, xylitol, erythritol, isomalt, lactitol, polyvinyl alcohol, or a mixture thereof, wherein the polyol concentration is from 200 mg/ml to 500 mg/ml,
c) a polymer which is polyvinylpyrrolidone having an average molecular weight from 20,000 to 1,500,000, and/or copovidone having an average molecular weight from 45,000 to 70,000, wherein the concentration of the polymer is from 1 mg/ml to 10 mg/ml,
d) water,
e) a buffer,
wherein the pH of the solution is from 4.0 to 7.5.

According to another preferred embodiment, the oral pharmaceutical solution according to the invention comprises from 8 mg/ml to 18 mg/ml of a pharmaceutically acceptable salt of cinacalcet, and a pharmaceutically acceptable carrier comprising
a) a mixture of propylene glycol and polyethylene glycol having an average molecular weight from 150 to 800, or a mixture of two polyethylene glycols having an average molecular weight from 150 to 800, wherein the concentration of the mixture is from 200 mg/ml to 600 mg/ml,
b) a polyol selected from maltitol, glycerol, mannitol, sorbitol or xylitol, or a mixture thereof, wherein the polyol concentration is from 200 mg/ml to 500 mg/ml,
c) a polymer which is polyvinylpyrrolidone having an average molecular weight from 20,000 to 1,500,000, and/or copovidone having an average molecular weight from 45,000 to 70,000, wherein the concentration of the polymer is from 1 mg/ml to 10 mg/ml,
d) water,
e) a buffer,
wherein the pH of the solution is from 4.0 to 7.5.

Preferably, the oral pharmaceutical solution according to the invention is free of surfactants.

The oral pharmaceutical solution according to the invention may also optionally contain additional excipients commonly used in preparing oral liquid compositions, such as an antimicrobial preservative, an antioxidant, a viscosity adjusting agent, a flavouring agent or a sweetener.

Examples of antimicrobial preservatives as referred to herein include but are not limited to sodium benzoate, benzoic acid, boric acid, sorbic acid and salts thereof, benzyl alcohol, parahydroxy benzoic acids and their alkyl esters, methyl, ethyl and propyl parahydroxy benzoates or their salts, or a mixture thereof.

Examples of antioxidants as referred to herein include but are not limited to sodium metabisulfite, butylated hydroxyanisole, butylated hydroxytoluene, ethylenediamine tetraacetic acid, ascorbic acid, α-tocopherof, propyl gallate, or a mixture thereof.

Examples of flavouring agents as referred to herein include but are not limited to fruit flavours such as orange, banana, strawberry, cherry, wild cherry, lemon and the like and other flavourings, such as cardamom, anise, mint, menthol, vanillin, bubble gum, or a mixture thereof.

Examples of sweeteners as referred to herein include but are not limited to sugars, i.e. carbohydrates, such as sucrose, glucose, dextrose, lactose, fructose and galactose as well as other sweetening agents known in the art such as sucralose, aspartame, acesulfame-K, thaumatin, mogroside, saccharin and salts thereof, sodium cyclamate, erythritol, glycyrrhizin, monosodium glycyrrhizinate, monoamonium glycyrrhizinate, or a mixture thereof.

It has also been found that the combination of the polyol and the polymer provides the oral solution of the invention with improved taste. This means that the composition of the present invention has an acceptable taste even if it does not further comprise a sweetener. Furthermore, when the composition further comprises a sweetener, its concentration can be kept at a lower level compared to the concentration that would be needed in the absence of the combination of the polyol and the polymer.

The oral pharmaceutical solution according to the invention is preferably supplied as a multidose preparation. Each dose from a multidose container, such as amber type III glass 50 or 100 ml bottles sealed with a suitable seal, such as a child resistant, tamper evident screw cap, can be administered by means of a device suitable for measuring the prescribed volume. The device is usually a spoon or a cup for volumes of 5 ml or multiples thereof, or an oral syringe for other volumes.

The oral pharmaceutical solution of the present invention may be prepared using methods well known in the art and using regular manufacturing equipment.

For example, it may be prepared using the following process:
The active substance (pharmaceutically acceptable salt of cinacalcet) and the excipients are weighed. Purified water is added to a vessel. The mixture of glycols and the polyol(s) are successively added to the vessel. The pharmaceutically acceptable salt of cinacalcet is then added under continuous stirring. The polymer is then dissolved into the mixture under stirring. An aqueous buffer solution, is prepared in a different vessel and is added under continuous stirring until the pharmaceutically acceptable salt of cinacalcet is completely dissolved. Preservative, if present, is also added under continuous stirring until complete dissolution. Flavour and the remaining excipients, if present, are successively added under continuous stirring, until complete dissolution. The pH of the solution is adjusted to the desired value by adding aqueous sodium hydroxide or hydrochloric acid solution. Finally, the volume is adjusted with purified water or with the buffer solution.

### EXAMPLES

The following examples show the influence of the proposed, according to the invention, aqueous carrier, on the palatability and solubility of cinacalcet hydrochloride.

### EXAMPLE 1

Sixteen liquid compositions were prepared through a manufacturing process, where a buffer solution of the desired pH value is prepared. Buffer corresponding to approximately 30% of the final batch size is added to a mixing vessel (main mixing vessel). Cinacalcet hydrochloride is added to the above solution under continuous stirring until it is completely dissolved. The polyol, glycols, povidone, if present and the selected sweetener are then added sequentially to the above solution under continuous stirring. The pH of the solution is adjusted, if necessary, to 6.0 by adding aqueous sodium hydroxide or hydrochloric acid solution. The volume is adjusted to the desired batch volume by adding buffer solution.

These compositions were studied in relation to their appearance and bitterness value according to the method described in paragraph 2.8.15 of Ph. Eur. - 10.0. According to this method the bitterness value of a liquid is the reciprocal of the dilution of quinine hydrochloride (the bitterness value of which is set at 200,000) that still has a bitter taste. An adult sensory panel of 8 persons was employed.

Table 1a shows three compositions which do not belong to the present invention (A, B, C), because they contain only one glycol and they do not contain a polymer, in comparison with a composition of the present invention (I).

**Table 1a - two (2) polyols / one (1) glycol / no polymer vs composition according to the invention**

| | **Excipients** | **mg/ml** | | | |
|---|---|---|---|---|---|
| | | **A** | **B** | **C** | **I** |
| **Active** | Cinacalcet HCl | 8 | 8 | 8 | 8 |
| **Polyol** | Glycerol | 200 | 200 | - | 200 |
| | Mannitol | - | 200 | 200 | - |
| | Sorbitol | 200 | - | 200 | 200 |
| **Glycol** | PG | 300 | - | 300 | 150 |
| | PEG 400 | - | 300 | - | 150 |
| **Polymer** | Povidone K-90 | - | - | - | 5 |
| **Sweetener** | Sucrose | 5 | - | - | 5 |
| | Glucose | - | 5 | 5 | - |
| | Fructose | 5 | 5 | 5 | 5 |
| | Taste | Bitter (10569) | Bitter (12569) | Not tested | Palatable (6098) |
| | Appearance (T= 2 hours) | White precipitate | White precipitate | White precipitate | Clear |

| | | | | | |
|---|---|---|---|---|---|
| PG; Propylene glycol, PEG; Polyethylene glycol | | | | | |

Table 1b shows three compositions which do not belong to the present invention (D, E, F), because they contain only one glycol, in comparison with a composition of the present invention (II).

**Table 1b - one (1) polyol / one (1) glycol / one (1) polymer vs composition according to the invention**

| | **Excipients** | **mg/ml** | | | |
|---|---|---|---|---|---|
| | | **D** | **E** | **F** | **II** |
| **Active** | Cinacalcet HCl | 8 | 8 | 8 | 8 |
| **Polyol** | Glycerol | - | - | 400 | - |
| | Sorbitol | 400 | 400 | - | 400 |
| **Glycol** | PG | 300 | - | 300 | 150 |
| | PEG 400 | - | 300 | - | 150 |
| **Polymer** | Povidone K-90 | 5 | 5 | 5 | 5 |
| **Sweetener** | Sucrose | 5 | - | - | 5 |
| | Glucose | - | 5 | 5 | - |
| | Fructose | 5 | 5 | 5 | 5 |
| | Taste | Bitter (9969) | Not tested | Not tested | Palatable (4999) |
| | Appearance (T= 2 hours) | White precipitate | White precipitate | White precipitate | Clear |

| | | | | | |
|---|---|---|---|---|---|
| PG; Propylene glycol, PEG; Polyethylene glycol | | | | | |

Table 1c shows three compositions which do not belong to the present invention (G, H, J), because they do not contain a polyol, in comparison with a composition of the present invention (III).

**Table 1c - no polyol / two (2) glycols / one (1) polymer vs composition according to the invention**

| | **Excipients** | **mg/ml** | | | |
|---|---|---|---|---|---|
| | | **G** | **H** | **J** | **III** |
| **Active** | Cinacalcet HCl | 8 | 8 | 8 | 8 |
| **Polyol** | Glycerol | - | - | - | 100 |
| | Mannitol | - | - | - | 100 |
| | Sorbitol | - | - | - | - |
| **Glycol** | PG | 300 | 200 | 300 | 200 |
| | PEG 400 | 300 | 300 | 200 | 300 |
| **Polymer** | Povidone K-90 | 5 | 5 | 5 | 5 |
| **Sweetener** | Sucrose | 5 | - | - | 5 |
| | Glucose | - | 5 | 5 | - |
| | Fructose | 5 | 5 | 5 | 5 |
| | Taste | Bitter (31256) | Bitter (29275) | Not tested | Palatable (7099) |
| | Appearance (T= 2 hours) | White precipitate | White precipitate | White precipitate | Clear |

| | | | | | |
|---|---|---|---|---|---|
| PG; Propylene glycol, PEG; Polyethylene glycol | | | | | |

Table 1d shows three compositions which do not belong to the present invention (J, K, L), because they contain only one glycol and they do not contain a polymer, in comparison with a composition of the present invention (IV).

**Table1d - one (1) polyol / one (1) glycol / no polymer vs composition according to the invention**

| | **Excipients** | **mg/ml** | | | |
|---|---|---|---|---|---|
| | | **J** | **K** | **L** | **IV** |
| **Active** | Cinacalcet HCl | 8 | 8 | 8 | 8 |
| **Polyol** | Glycerol | 400 | - | - | - |
| | Mannitol | - | 400 | - | 400 |
| | Sorbitol | - | - | 400 | - |
| **Glycol** | PG | 300 | - | 300 | 150 |
| | PEG 400 | - | 300 | - | 150 |
| **Polymer** | Povidone K-90 | - | - | - | 5 |
| **Sweetener** | Sucrose | - | - | - | 5 |
| | Glucose | - | 5 | 5 | - |
| | Fructose | 5 | 5 | 5 | 5 |
| | Taste | Bitter (12257) | Not tested | Not tested | Palatable (5192) |
| | Appearance (T= 2 hours) | White precipitate | White precipitate | White precipitate | Clear |

| | | | | | |
|---|---|---|---|---|---|
| PG; Propylene glycol, PEG; Polyethylene glycol | | | | | |

The above results clearly demonstrate that the combination of excipients according to the present invention results in compositions in which the active ingredient is dissolved. Furthermore, the compositions exhibit good taste. Conversely, the absence of at least one of the excipients as defined in the present invention results in compositions in which a precipitate is formed and/or exhibit an unacceptable taste.

### EXAMPLE 2

Six liquid compositions were studied in relation to their appearance and bitterness value according to the method described in paragraph 2.8.15 of Ph. Eur. - 10.0. According to this method the bitterness value of a liquid is the reciprocal of the dilution of quinine hydrochloride (the bitterness value of which is set at 200,000) that still has a bitter taste. An adult sensory panel of 8 persons was employed.

These compositions were prepared through a manufacturing process, where purified water is added into a vessel. The mixture of glycols and the polyol(s), were successively added into the vessel. Cinacalcet hydrochloride was then added under continuous stirring. The polymer was dissolved into the mixture under stirring. A pH buffer solution, prepared in a different vessel, was added under continuous stirring until cinacalcet hydrochloride was completely dissolved. Preservative was added under continuous stirring until complete dissolution. The sweetening agent was then added under continuous stirring, until complete dissolution. The pH of the solution was adjusted with a quantity of the buffer solution to the desired value. Finally, the volume was adjusted with a quantity of the buffer solution.

**Table 2a:**

| | **Compositions (mg/ml)** | | |
|---|---|---|---|
| **Ingredient** | **V** | **VI** | **VII** |
| Cinacalcet HCl | 8 | 12 | 14 |
| Propylene glycol | 150 | 150 | 200 |
| Polyethylene glycol-400 | 250 | 300 | 300 |
| Liquid sorbitol | 350 | 350 | 400 |
| Povidone K-90 | 3 | 4 | 5 |
| Sucralose | 5 | 5 | 10 |
| Sodium methyl paraben | 0.8 | 0.8 | 0.8 |
| Citric acid / sodium citrate buffer solution | pH 6.0 | | |
| Citric acid / sodium citrate buffer solution | q.s to 1.0 mL | | |
| Taste of solution (Bitterness value) | Palatable (1212) | Palatable (1393) | Palatable (3101) |
| Appearance of solution (T=0) | Clear | Clear | Clear |
| Appearance of solution (T= 3 months) | Clear | Clear | Clear |

**Table 2b:**

| | **Compositions (mg/ml)** | | |
|---|---|---|---|
| **Ingredient** | **VIII** | **IX** | **X** |
| Cinacalcet HCl | 8 | 10 | 12 |
| Propylene glycol | 200 | 150 | - |
| Polyethylene glycol-400 | 250 | 300 | 200 |
| Polyethylene glycol-600 | - | - | 200 |
| Mannitol | 300 | 350 | 350 |
| Povidone K-25 | 4 | 4 | 6 |
| Sucralose | 5 | 5 | 10 |
| Sodium methyl paraben | 0.8 | 0.8 | 0.8 |
| Citric acid / sodium citrate buffer solution | pH 6.0 | | |
| Citric acid / sodium citrate buffer solution | q.s to 1.0 mL | | |
| Taste of solution (Bitterness value) | Palatable (3290) | Palatable (3960) | Palatable (7106) |
| Appearance of solution (T=0) | Clear | Clear | Clear |
| Appearance of solution (T= 3 months) | Clear | Clear | Clear |

**Table 2c:**

| | **Compositions (mg/ml)** | | |
|---|---|---|---|
| **Ingredient** | **XI** | **XII** | **XIII** |
| Cinacalcet HCl | 10 | 10 | 10 |
| Propylene glycol | 150 | 150 | 150 |
| Polyethylene glycol-400 | 250 | 250 | 250 |
| Mannitol | 350 | 350 | 350 |
| Povidone K-25 | 2 | - | |
| Copovidone K25-31 | 2 | 6 | 6 |
| Sucralose | - | 2 | - |
| Sodium methyl paraben | 0.8 | 0.8 | 0.8 |
| Citric acid / sodium citrate buffer solution | pH 6.0 | | |
| Citric acid / sodium citrate buffer solution | q.s to 1.0 mL | | |
| Taste of solution (Bitterness value) | Palatable (6990) | Palatable (5160) | Palatable (7966) |
| Appearance of solution (T=0) | Clear | Clear | Clear |

## Claims

1. An oral pharmaceutical solution comprising a pharmaceutically acceptable cinacalcet salt as active ingredient and a pharmaceutically acceptable carrier comprising
a) a mixture of propylene glycol and a polyethylene glycol having an average molecular weight from 150 to 800, or a mixture of two polyethylene glycols having an average molecular weight from 150 to 800,
b) a polyol selected from glycerol, sorbitol, mannitol, maltitol, xylitol, erythritol, isomalt, lactitol, polyvinyl alcohol, or mixtures thereof,
c) a polymer which is polyvinylpyrrolidone having an average molecular weight from 20,000 to 1,500,000, and/or copovidone having an average molecular weight from 45,000 to 70,000
d) water and
e) a buffer,
wherein the pH of the solution is from 4.0 to 7.5.

2. The oral pharmaceutical solution according to claim 1, wherein the polyethylene glycol is selected from polyethylene glycol 200, polyethylene glycol 300, polyethylene glycol 400, polyethylene glycol 540, or polyethylene glycol 600.

3. The oral pharmaceutical solution according to claim 1 or 2, wherein the polyol is selected from glycerol, sorbitol, mannitol, maltitol xylitol, or a mixture thereof.

4. The oral pharmaceutical solution according to any one of the preceding claims, wherein the polymer is selected from povidone K25, povidone K90 or copovidone K25-31.

5. The oral pharmaceutical solution according to any one of the preceding claims, wherein the polyethylene glycol is polyethylene glycol 400, the polyol is sorbitol and the polymer is povidone K90.

6. The oral pharmaceutical solution according to any one of the preceding claims, wherein the concentration of the pharmaceutically acceptable cinacalcet salt in the solution is from 8 mg/ml to 18 mg/ml.

7. The oral pharmaceutical solution according to any one of the preceding claims, wherein the concentration of the pharmaceutically acceptable cinacalcet salt in the solution is from 10 mg/ml to 16 mg/ml.

8. The oral pharmaceutical solution according to any one of the preceding claims, wherein the concentration of the pharmaceutically acceptable cinacalcet salt in the solution is from 12 mg/ml to 14 mg/ml.

9. The oral pharmaceutical solution according to any one of the preceding claims, wherein the concentration of the mixture of propylene glycol and polyethylene glycol, or of the mixture of two polyethylene glycols in the solution is from 200 to 600 mg/ml, the polyol concentration in the solution is from 200 mg/ml to 500 mg/ml and the concentration of the polymer in the solution is from 1 mg/ml to 10 mg/ml.

10. The oral pharmaceutical solution according to any one of the preceding claims, wherein the concentration of the mixture of propylene glycol and polyethylene glycol, or of the mixture of two polyethylene glycols in the solution is from 250 to 550 mg/ml, the polyol concentration in the solution is from 200 mg/ml to 450 mg/ml and the concentration of the polymer in the solution is from 2 mg/ml to 9 mg/ml.

11. The oral pharmaceutical solution according to any one of the preceding claims, wherein the concentration of the mixture of propylene glycol and polyethylene glycol, or of the mixture of two polyethylene glycols in the solution is from 300 to 500 mg/ml, the polyol concentration in the solution is from 200 mg/ml to 400 mg/ml and the concentration of the polymer in the solution is from 3 mg/ml to 8 mg/ml.

12. The oral pharmaceutical solution according to any one of the preceding claims, wherein the pH of the solution is from 5.0 to 6.5.

13. The oral pharmaceutical solution according to any one of the preceding claims, wherein the pharmaceutically acceptable cinacalcet salt is cinacalcet hydrochloride.

14. The oral pharmaceutical solution according to any one of the preceding claims, wherein the solution does not comprise a surfactant.

15. The oral pharmaceutical solution according to any one of the preceding claims, wherein the solution further comprises a sweetener.
